# EUROPEAN PATENT SPECIFICATION

(11) **EP 2 729 128 B1**
(45) Date of publication and mention of the grant of the patent: **27.07.2016**
(21) Application number: 12732668.4
(22) Date of filing: 06.07.2012
(51) Int. Cl.: A61K 9/16, A61K 31/34, A61K 45/06, A61K 47/38, A61K 9/20, A61K 31/635, A61P 31/18

(54) **DARUNAVIR FORMULATIONS**
DARUNAVIRFORMULIERUNGEN
FORMULATIONS DE DARUNAVIR

(30) Priority: 07.07.2011 EP 11173066
(43) Date of publication of application: 14.05.2014
(73) Proprietor: Janssen Sciences Ireland UC, Little Island, County Cork (IE)
(72) Inventor: DELAET, Urbain Alfons C, B-2490 Balen (BE); HEYNS, Philip Erna H, B-2350 Vosselaar (BE); JANS, Eugeen Maria Jozef, B-2450 Meerhout (BE)
(74) Representative: Daelemans, Frank F.R.
(86) International application number: PCT/EP2012/063242
(87) International publication number: WO 2013/004816

(56) References cited:
- WO-A2-2009/000853
- WO-A2-2009/013356
- WO-A2-2009/081174
- WO-A2-2011/048604

## Description

### Field of the Invention

This invention relates to solid oral dosage forms of the HIV inhibitor darunavir and combination formulations thereof.

### Background of the Invention

The treatment of Human Immunodeficiency Virus (HIV) infection, known as cause of the acquired immunodeficiency syndrome (AIDS), remains a major medical challenge. HIV is able to evade immunological pressure, to adapt to a variety of cell types and growth conditions and to develop resistance against currently available drug therapies. The latter include nucleoside reverse transcriptase inhibitors (NRTIs), non-nucleoside reverse transcriptase inhibitors (NNRTIs), nucleotide reverse transcriptase inhibitors (NtRTIs), HIV-protease inhibitors (PIs) and the more recent fusion inhibitors.

Although effective in suppressing HIV, each of these drugs, when used alone, is confronted with the emergence of resistant mutants. This led to the introduction of combination therapy of several anti-HIV agents usually having a different activity profile. In particular the introduction of "HAART" (Highly Active Anti-Retroviral Therapy) resulted in a remarkable improvement in anti-HIV therapy, leading to a large reduction in HIV-associated morbidity and mortality. Current guidelines for antiretroviral therapy recommend such triple combination therapy regimen even for initial treatment. However, none of the currently available drug therapies is capable of completely eradicating HIV. Even HAART may face the emergence of resistance, often due to non-adherence and non-persistence with antiretroviral therapy. In these cases HAART can be made effective again by replacing one of its components by one of another class. If applied correctly, treatment with HAART combinations can suppress the virus for many years, up to decades, to a level where it no longer can cause the outbreak of AIDS.

Because of their pharmacokinetic properties and the need to keep plasma levels above a minimum level, currently used anti-HIV drugs require frequent administration of relatively high doses. The number and/or volume of dosage forms that need to be administered are commonly referred to as the "pill burden". A high pill burden is undesirable for many reasons, such as the frequency of intake, often combined with the inconvenience of having to swallow large dosage forms, as well as the need to store and transport a large number or volume of pills. A high pill burden increases the risk of patients not taking their entire dose, thereby failing to comply with the prescribed dosage regimen. As well as reducing the effectiveness of the treatment, this also leads to the emergence of viral resistance. The problems associated with a high pill burden are multiplied where a patient must take a combination of different anti-HIV agents or agents in combination with a so called booster to improve pharmacokinetic properties.

Providing high dosage forms that have a relatively small size contributes to the convenience of intake and therefore also helps to overcome problems of pill burden.

Therefore, it would be desirable to provide HIV inhibitory therapy that reduces pill burden in that it involves the administration of dosage forms of a practical size and additionally does not require frequent dosing.

One class of HIV drugs that is used in HAART is that of the PIs amongst which is darunavir (TMC114), approved in the U.S., the E.U. and a number of other countries and available under the trade name Prezista™. darunavir, currently marketed in the form of darunavir monoethanolate, has the following chemical name: [(1*S*,2*R*)-3-[[(4-aminophenyl)sulfonyl](2-methylpropyl)amino]-2-hydroxy-1-(phenylmethyl)-propyl]-carbamic acid (3*R*,3a*S*,6a*R*)-hexahydrofuro[2,3-*b*]furan-3-yl ester monoethanolate. Its molecular formula is C₂₇H₃₇N₃O₇S•C₂H₅OH, with a molecular weight of 593.73, and the following chemical structure: 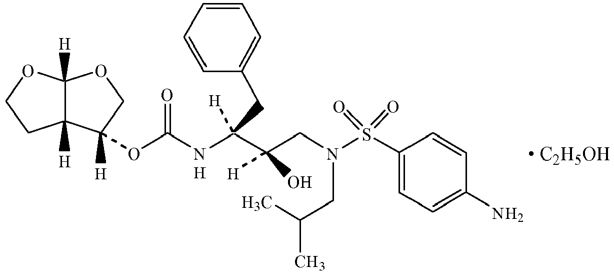 darunavir as well as processes for its preparation are disclosed in EP 715618, WO99/67417, US 6,248,775, and in Bioorganic and Chemistry Letters, Vol. 8, pp. 687-690, 1998, "Potent HIV protease inhibitors incorporating high-affinity P₂-ligands and (R) (hydroxyethylamino)sulfonamide isostere".

Improved combination formulations of darunavir with pharmacokinetic boosters, e.g. cytochrome P₄₅₀ inhibitors, are disclosed in WO03/049746.

WO 2011/048604 A2 discloses darunavir formulations comprising granules prepared by wet granulation of darunavir, hypromellose and several other excipients.

Because high darunavir dosage forms are inevitably large in size, higher dose or combination dosage forms would take a size that surpasses the convenience barrier. In order to reduce pill-burden it would be desirable to achieve a dosage form with an increased weight % of darunavir per dosage form. This would facilitate either the generation of a higher dose tablet, or a reduction in size of the present dose tablets. It would be additionally desirable to combine darunavir, especially high dosages of darunavir, and a pharmacokinetic booster agent e.g. ritonavir in one dosage form.

A darunavir tablet containing 600 mg of active ingredient and having a total weight of 1250 mg per tablet is disclosed in WO2009/013356. The oral dosage forms are formed by direct compression of the ingredients.

Higher dose darunavir formulations, dose-proportionally derived from the currently marketed 600-mg tablet, were not deemed desirable for use by patients because of their large size.

Furthermore, the direct compression method led to inferior results when increasing the percentage of darunavir in the formulation. Inferior results are obtained due to limited gliding and flowing capacity of such a formulation. This is also the case when other actives are added to the formulation.

The present invention is based on the unexpected finding that a high weight % load of darunavir per dosage form is facilitated by the granulation of darunavir before formulation.

Granulation of darunavir according to the present invention thus facilitates a high loading of darunavir in a single dosage form (> 80 % (w/w)) or the combination of darunavir with other active ingredients and still having an acceptable size of the dosage form.

The present invention thus provides anti-HIV therapy involving the administration of darunavir dosage forms of acceptable size, potentially as a combination formulation, thereby requiring less frequent dosing. Hence, present dosage forms are beneficial in terms of pill burden and drug compliance of the patient.

### Summary of the Invention

In one aspect the invention relates to a darunavir granulate composition consisting of darunavir or a pharmaceutically acceptable salt or solvate thereof, Hypromellose and any residual water from the granulation.

Preferably, the darunavir is present in the form of its ethanolate and the Hypromellose is Hypromellose 2910 15 mPa.s.

In another aspect, the invention relates to an oral dosage form comprising about 0.4 to 0.6% by weight (w/w) of a lubricant, about 2 to 4 % by weight (w/w) of a disintegrant, microcrystalline cellulose, and about 50 to 90 % by weight (w/w) of a darunavir granulate according to claims 1 or 2, the core being optionally coated with a film coating.

In yet another aspect, the invention relates to a process for preparing an oral dosage form according to the invention comprising the steps of:
- Providing granulated darunavir by; mixing water and Hypromellose, spraying this first mixture on a powder of darunavir or a pharmaceutically acceptable salt or solvate thereof, and drying the so obtained darunavir granulate
- Providing a second mixture comprising microcrystalline cellulose, and a disintegrant,
- Adding granulated darunavir to the mixture and subsequent dry-blending
- Adding a lubricant and mixing until homogeneous,
- Compressing the mixture to provide the oral dosage form, said oral dosage form then being optionally film-coated.

In yet another aspect, the present invention relates to an oral dosage form according to the invention for use in medicine, more specifically for use in the treatment of HIV infections.

Additionally, a method for the treatment of an HIV infection in a subject is described herein. Said method comprises administering to the subject an effective amount of an oral dosage form according to the invention.

By making use of this granulate, the weight percentage darunavir can be increased per dosage form, thus generating oral dosage forms with a high dose of free from equivalent of darunavir (e.g. 800mg). Additionally, the size and weight of existing dosage forms (e.g. 400 or 600mg) can be reduced by about 25%.

Advantageously, the solid oral dosage forms can optionally comprise additional active ingredients such as pharmacokinetic boosters, e.g. ritonavir and still be of an acceptable size. The size of the dosage forms of the invention, i.e. the total weight of the dosage forms, should be below a limit of convenience which is below the size at which a number of patients starts having difficulty taking in the dosage form.

The oral dosage forms of the present invention preferably are tablets.

As used herein, the term "darunavir" is meant to comprise the base form, any pharmaceutically acceptable acid addition salt thereof, as well as any pharmaceutically acceptable solvate thereof. The pharmaceutically acceptable addition salts as mentioned hereinabove the therapeutically active non-toxic acid addition salt forms, which darunavir is able to form. In one embodiment the term "darunavir" is meant to comprise the base form, as well as any pharmaceutically acceptable solvate thereof.

The term pharmaceutically acceptable solvate comprises the hydrates and the solvent addition forms that darunavir can form. Examples of such forms are e.g. hydrates, alcoholates, e.g. methanolates, ethanolates and propanolates, and the like. Particular solvates are the ethanolate, e.g. the monoethanolate.

As used herein the term "free-form equivalent" refers to that quantity of darunavir whether present in free form (or base form), or as salt or solvate, that corresponds to a given quantity of free form darunavir. For example 650 mg of darunavir monoethanolate corresponds to 600 mg of free-form equivalent darunavir.

For application in adults, high quantities of the active ingredients may be used. In such instance, the dosage forms of the invention contain from about 500 to about 900 mg, in particular from about 600 mg to about 800 mg, for example about 800 mg, of free-form equivalent darunavir per unit of the dosage form.

The darunavir in the dosage forms of the invention is added to the formulation process in the form of a darunavir granulate composition consisting of darunavir or a pharmaceutically acceptable salt or solvate thereof, Hypromellose and any residual water from the granulation.

Preferably, the darunavir is present in the form of its ethanolate and the Hypromellose is Hypromellose 2910 15 mPa.s.

The amount of darunavir in the granulate composition may be in the range from about 95% to about 100%, in particular about 97% to about 99.9%, or about 98% to about 99% , by weight relative to the total weight of the granulate composition comprising darunavir and Hypromellose 2910 15 mPa.s. The granulate composition may additionally contain residual water that is not completely removed during processing.

The average particle size of the granulate is between 100 and 500 µm, more preferably from 150 to 400 µm and even more preferably about 300 µm.

As used herein, the term average particle size has its conventional meaning as known to the person skilled in the art and can be measured by art-known particle size measuring techniques such as, for example, sedimentation field flow fractionation, photon correlation spectroscopy, laser diffraction or disk centrifugation. The average particle sizes mentioned herein may be related to weight distributions of the particles. In that instance, by "an average particle size of about 150 µm" it is meant that at least 50% of the weight of the particles have a particle size of less than average of 50 µm, and the same applies to the other particle sizes mentioned. In a similar manner, the average particle sizes may be related to volume distributions of the particles but usually this will result in the same or about the same value for the average effective particle size.

Granulation of darunavir preferably is performed in a fluid-bed granulator. Preferably, darunavir is granulated by using Hypromellose. More preferably, Hypromellose 2910 15 mPa.s is used. According to the present invention, darunavir is granulated without any filler or other excipients before formulation of the tablet core.

Preferably, the oral dosage forms according to the present invention will comprise one or more other active ingredients. An active ingredient is a compound with a pharmacokinetic or pharmacological effect. Non limiting examples of such an active compound are cytochrome P₄₅₀ inhibitors or HIV inhibitors. The latter preferably include HIV inhibitors of other classes, in particular an NRTI, or NNRTI, but also a fusion inhibitor. HIV inhibitors that may be co-administered by preference are those used in HAART combinations.

Preferably, the oral dosage forms according to the present invention will comprise a pharmacokinetic booster such as a cytochrome P₄₅₀ inhibitor. Suitable examples of such a booster are selected from the group comprising ritonavir, indinavir, nelfinavir, saquinavir, amprenavir, lopinavir, lasinavir, palinavir, telinavir, tipranavir, mozenavir, atazanavir and pharmaceutically acceptable salts and esters thereof. More in particular, said inhibitor may be selected from the group comprising, ritonavir, amprenavir, nelfinavir or a pharmaceutically acceptable salt or ester thereof.

Oral dosage forms according to the present invention will preferably comprise pharmaceutically acceptable carriers and excipients. Such inactive ingredients are added to help hold the tablet together and give it strength, among others binders, fillers disintegrant glidants and lubricants.

A wide variety of binders may be used, some common ones including lactose, dibasic calcium phosphate, sucrose, corn (maize) starch, microcrystalline cellulose and modified cellulose (for example hydroxymethyl cellulose). Other such materials are silicon dioxide, titanium dioxide, alumina, talc, kaolin, powdered cellulose, as well as soluble materials such as mannitol, urea, sucrose, lactose, dextrose, sodium chloride, and sorbitol. Such agents may sometimes also be referred to as "fillers".

Microcrystalline cellulose that can be used comprises the Avicel™ series of products available from FMC BioPolymer, in particular AvicelPH 105® (20 µm), Avicel PH 101® (50 µm), Avicel PH 301® (50 µm);
the microcrystalline cellulose products available from JRS Pharma, in particular Vivapur® 105 (20 µm), Vivapur® 101 (50 µm), Emcocel® SP 15 (15 µm), Emcocel® 50M 105 (50 µm), Prosolv® SMCC 50 (50 µm);
the microcrystalline cellulose products available from DMV, in particular Pharmacel®105 (20 µm), Pharmacel®101 (50 µm);
the microcrystalline cellulose products available from Blanver, in particular Tabulose (Microcel)®101 (50 µm), Tabulose (Microcel)®103 (50 µm);
the microcrystalline cellulose products available from Asahi Kasei Corporation, such as Ceolus® PH-F20JP (20 µm), Ceolus® PH-101 (50 µm), Ceolus® PH-301 (50 µm), Ceolus® KG-802 (50 µm).

A particularly preferred microcrystalline cellulose is Ceolus® KG-802, average particle size (50 µm). Additional characteristics of Ceolus® KG-802 are a bulk density of about 0.2(g/cm³) and an angle of repose of about 49°.

The average particle size of the Microcrystalline cellulose may be in the range of from 5 µm to 60 µm, in particular from 10 µm to 50 µm, e.g. about 20 µm.

In addition to the presence of any of the above indicated ingredients, the tablet formulation according to the invention contains a lubricant. This provides a formulation which avoids manufacturing problems such as tablet sticking when the drug product blend is compressed into tablets.

The lubricant is preferably magnesium stearate and is generally present in an amount of 0.4 to 0.6 % w/w, particularly about 0.5% w/w.

The tablet formulation also contains a disintegrant to aid disintegration and dissolution of the formulation upon administration to the patients. The preferred disintegrant is crospovidone, namely a synthetic homopolymer of cross-linked N-vinyl-2-pyrrolidone available commercially as Polyplasdone XL-10 and is preferably present in an amount of 1 to 4% w/w, especially about 3% w/w. Other disintegrants which may be used include croscarmellose sodium (sodium salt of cross-linked carboxymethylcellulose), available commercially as Acdisol.

The above tablet formulations can be used to make tablet cores in conventional manner for example by initially dry blending the ingredients, that preferably having been sieved. Subsequently, the lubricant is added to the dry-blended mixture for final dry-blending of the total tablet core blend, which is then compressed into tablets having the desired size and weight.

For taste-masking and cosmetic reasons the tablet cores according to the invention are generally provided with a film coating for example an Opadry film-coating, which is generally used in an amount of about 4% w/w based on the tablet core. Different coloring agents may be used in the film coating in order to differentiate between tablet strengths.

The coating can be applied to the core in coating suspension for example in purified water, followed by drying of the coated cores.

The administration of a dosage form in accordance with the present invention may suffice to treat HIV infection although it may be recommendable to co-administer other HIV inhibitors. The latter preferably include HIV inhibitors of other classes, in particular an NRTI, or NNRTI, but also a fusion inhibitor can be added. HIV inhibitors that may be co-administered by preference are those used in HAART combinations.

In certain instances, the treatment of HIV infection may be limited to only the dosage form of the invention, without co-administration of further HIV inhibitors. This option may be recommended, for example, where the viral load is relatively low, e.g. where the viral load (represented as the number of copies of viral RNA in a specified volume of serum) is below about 200 copies/ml, in particular below about 100 copies/ml, more in particular below 50 copies/ml, specifically below the detection limit of the virus. This type of monotherapy may be applied after initial treatment with a combination of HIV drugs, such as any of the HAART combinations during a certain period of time until the viral load in blood plasma reaches the afore mentioned low viral level.

In a further aspect the present invention relates to the use of a dosage form in accordance with the invention, for the manufacture of a medicament for maintenance therapy of a subject infected with HIV. The present invention also relates to the use of a dosage form in accordance with the invention, for the manufacture of a medicament for treating a subject infected with HIV, wherein the dosage form is combined with two different NRTIs or NNRTIs.

As used herein the term "treatment of HIV infection" relates to a situation of the treatment of a subject being infected with HIV. The term "subject" in particular relates to a human being.

The doses of darunavir and optional other active compounds in the dosage forms of the invention are selected so as to keep the blood plasma concentration of darunavir above the minimum blood plasma level between two administrations. The term "minimum blood plasma level" in this context refers to the lowest efficacious blood plasma level, the latter being that blood plasma level of active that provides effective treatment of HIV. The plasma levels of anti-HIV compounds should be kept above these threshold blood plasma levels because at lower levels the drugs may no longer be effective thereby increasing the risk of mutations.

The dosage forms of the present invention provide effective treatment of HIV infection in that the viral load is reduced while keeping viral replication suppressed. The limited number of drug administrations adds to the patients' compliance with the prescribed therapy.

As used herein, the word "substantially" does not exclude "completely" *e.g.* a composition which is "substantially free" from Y may be completely free from Y. Where necessary, the word "substantially" may be omitted from the definition of the invention. The term "about" in connection with a numerical value is meant to have its usual meaning in the context of the numerical value. Where necessary the word "about" may be replaced by the numerical value ±10%, or ±5%, or ±2%, or ±1%.

### Examples

### General

Excipients used throughout the examples are listed in Table 1.

**Table 1: Excipients**

| | |
|---|---|
| Excipient | Reference name |
| PROSOLV^{®} SMCC HD90 | HD90 |
| Hypromellose 2910 15 mPa.s | Methocel E15LV premium |
| Colloidal Anhydrous Silica^{a} | Cab-O-Sil M5P^{b} |
| Crospovidone | Polyplasdone XL-10 |
| Magnesium Stearate | Vegetal, type 5712 |
| Coating powder brick red | Opadry II brick red 85F250001 |

| | |
|---|---|
| ^{a} Colloidal Anhydrous Silica is alternately known as Colloidal Silicon Dioxide ^{b} Alternative is Aerosil 200 from Degussa | |

The film coating, combined with debossing and differences in tablet size, aids in the differentiation of the tablet strengths. A secondary function of the film coating is taste masking.

The excipients used in Opadry II red 85F250001 are listed in Table 2.

**Table 2: Composition for Coating powder brick red (Opadry II red 85F250001)**

| Component | Composition (w/w) |
|---|---|
| Polyvinyl alcohol | 40.00 |
| Polyethylylene glycol 3350 | 20.20 |
| Talc | 14.80 |
| Titanium dioxide | 3.26 |
| Iron Oxide Red | 20.01 |
| Iron Oxide Yellow | 1.21 |
| Iron Oxide Black | 0.52 |

### Example 1: darunavir granulation

### 1: Granulation

A high dose formulation, e.g. 800-mg darunavir formulation, dose-proportionally derived from the currently marketed 600-mg tablet, was not perceived as suitable for use by patients because of its large size. Furthermore, direct compression of an 800 mg formulation proved not possible due to severely limited gliding and flowing capacity. The formulations studied are shown in Table 3.

**Table 3: Formulations used in concept feasibility testing**

| Ingredients | A | | B | | C | |
|---|---|---|---|---|---|---|
| | mg/tab | % | mg/tab | % | mg/tab | % |
| darunavir | 867.28 | 69.38 | 867.28 | 72.27 | 867.28 | 72.27 |
| MCC^{a} | -- | -- | 287.12 | 23.93 | -- | -- |
| HPMC 2910 15 mPa.s | -- | -- | -- | -- | 24.00 | 2.00 |
| Purified water^{b} | -- | -- | 1043µl | -- | 600µl | -- |
| Prosolv HD90 | 337.08 | 26.97 | -- | -- | 266.72 | 22.23 |
| Crospolyvidone | 25.01 | 2.00 | 36.00 | 3.00 | 36.00 | 3.00 |
| Colloidal anhydrous silica | 11.38 | 0.91 | 3.60 | 0.30 | -- | -- |
| Magnesium stearate | 9.25 | 0.74 | 6.00 | 0.50 | 6.00 | 0.50 |
| **Total** | 1250 | 100 | 1200 | 100 | 1200 | 100 |

| | | | | | | |
|---|---|---|---|---|---|---|
| ^{a} MCC = Microcrystalline Cellulose (Avicel PH101) ^{b} Purified water does not appear in the final product | | | | | | |

### Direct compression formulation A:

All ingredients, except magnesium stearate, were sieved over a stainless steel screen of 0.95mm and blended for 10 minutes using a lab-scale planetary mixer. In a second blending step, the magnesium stearate was sieved and mixed for 5 minutes. The blend was not compressed, because of the bad flowability (angle of repose).

### Wet granulation formulation B:

The powders of the internal phase (API/MCC) were sieved over a stainless steel screen with 0.95 mm sieve openings and transferred into the granulation insert of the fluid bed granulator GPCG1.

The purified water (without binder) was sprayed on the powder mixture. The process conditions for the granulation are reported in the table below.

**Table 4 : Granulation conditions (B)**

| | Mixing/heating | Granulation | Drying |
|---|---|---|---|
| Air flow | 63 > 64 m³/h | 64 < > 112 m³/h | 108 > 65 m³/h |
| Spray rate | - | 13 -> 23 g/min. | - |
| Atomizing air flow | 1.0 bar | 1.0 bar | 1.0 bar |
| Inlet air temperature | 60°C (set) | 45°C (set) | 60 > 70°C (set) |
| | | 60 > 45°C (actual) | 45 < > 77°C (actual) |
| Outlet air temperature | 24 > 30°C | 29 > 24°C | 23 > 38°C |

The dried granules and the excipients of the external phase were sieved (0.95 mm) and blended for 10 min. In a second step, the magnesium stearate was sieved, added and blended for 5 min. The granulate after sieving was tested for granulometrics and LOD.

This final mixture was compressed at different compression forces (750 → 2000 kg), using a single punch tablet press. The obtained tablets (nom. weight 1200 mg, punch AC27/42: 20 mm x 9.5 mm, radius 3 mm, oblong shape) were analyzed for hardness, disintegration time and dissolution.

### Wet granulation formulation C:

The API was sieved over a stainless steel screen with 0.95 mm sieve openings and transferred into the granulation insert of the fluid bed granulator GPCG1.

The binder solution (HPMC 15 cps 4% solution in water) was sprayed on the powder mixture. The process conditions for the granulation are reported in the table below.

**Table 5 : Granulation conditions GPCG1 (C)**

| | Mixing/heating | Granulation | Drying |
|---|---|---|---|
| Air flow | 60 m³/h | 60 < > 113 m³/h | 93 > 90 m³/h |
| Spray rate | - | 20 g/min | - |
| Atomizing air flow | 1.0 bar | 1.0 bar | 1.0 bar |
| Inlet air | 60°C (set) | 45< >55°C (set) | 60°C (set) |
| temperature | | 51< >56°C (actual) | 57< >68°C (actual) |
| Outlet air temperature | 24 > 31 °C | 31 > 24°C | 25 > 38°C |

The dried granules and the excipients of the external phase were sieved (0.95 mm) and blended for 10 min. In a second step, the magnesium stearate was sieved, added and blended for 5 min.

Tablet characteristics of the compression mixtures (B and C are shown in Table 6. The Direct Compression concept A was not compressed, because of insufficient flowability (high angle of repose) of the blend. Tablet hardness was measured according to industry standard.

**Table 6 : Compression data and tablet characteristics**

| | **B** | | | | | |
|---|---|---|---|---|---|---|
| Comp. force | 750 kg | 1000 kg | 1250 kg | 1500 kg | 1750 kg | 2000 kg |
| Blend Flow | Tendency towards rat holing in hopper | | | | | |
| Aspect | Tablet splitting - lack of binding | | | | | OK |
| Hardness - daN | | | NE¹ | | | 18.0 |
| Disint. time - sec | | | NE | | | 134 |

| | **C** | | | | | |
|---|---|---|---|---|---|---|
| Comp. force | 750 kg | 1000 kg | 1250 kg | 1500 kg | 1750 kg | 2000 kg |
| Blend Flow | Good flow (out of hopper) | | | | | |
| Aspect | OK (no defects) | | | | | |
| Hardness - daN | 8.8 | 11.9 | 14.6 | 15.6 | 19.4 | 19.0 |
| Disint. time - ' " | 2'11" | 3'13" | 6'18" | 15'34" | 21'29" | 23'23" |

| | | | | | | |
|---|---|---|---|---|---|---|
| ¹ NE = not executed | | | | | | |

Concept (C), in which the darunavir is granulated solely with an aqueous HPMC 15 mPa.s binder solution and Prosolv HD90 filler material is added extra-granularly (i.e., in the final dry mixture), provided a superior process.

### 2: Darunavir 800 mg representative formulation

Based on the superior process including granulation, a representative oral dosage form comprising 800 mg free from equivalent of darunavir was formulated. The qualitative and quantitative composition of such a representative oral dosage form is provided in Table 7.

**Table 7: Representative darunavir (TMC114) 800-mg Tablet**

| | 800 mg | |
|---|---|---|
| Component | (mg/tablet) | (% wt) |
| Core Tablet | | |
| darunavir Ethanolate | 867.28^{a} | 78.84 |
| Hypromellose 2910 15 mPa.s | 13.20 | 1.20 |
| Purified water ^{b} | 330.00 µL | 0.00 |
| Silicified Mycrocrystalline Cellulose^{c} | 177.72 | 16.16 |
| Crospovidone | 33.00 | 3.00 |
| Colloidal Anhydrous Silica | 3.30 | 0.30 |
| Magnesium Stearate | 5.50 | 0.50 |
| Core Tablet Weight | 1100.00 | 100.00 |
| Film Coating | | |
| Coating powder brick red | 44.00 | 4.00 |
| Purified Water ^{b} | 176.00 µL | 0.00 |
| Total Tablet Weight | 1144.00 | 104.00 |

| | | |
|---|---|---|
| ^{a} Quantity of darunavir ethanolate equivalent to 800 mg of darunavir. ^{b} Purified Water does not appear in the final product. ^{c} A commercially available ('Prosolv HD90'), spray-dried mixture consisting of 98% (w/w) microcrystalline cellulose and 2% (w/w) colloidal silicon dioxide, individually meeting compendial requirements. | | |

### 3: Large scale manufacturing process according to the present invention

Several large scale badges were produced according to the specifications below. *Reparation of the 4% binder solution:*
- 1/3 of total quantity of purified water was warm up until 75 - 85°C.
- Hypromellose 2910 15mPa.s was added while mixing with strong vortex.
- After mixing for 10 - 20 min, the rest of (cold) purified water was added, while mixing with vortex for 5 - 10 minutes. The creation of foam was avoided by pouring the water slowly along the wall of the vessel.
- The solution was cooled and de-aerated until is clear and the temperature was = or < 30°C.
- Gentle mixing was applied for 1 - 2 min before the start of the granulation

### Wet granulation conditions (on GPCG-30 granulator)

Darunavir was transferred into the granulation insert of the fluid bed granulator GPCG-30 and pre-warmed. The binder solution (HPMC 15 cps 4% solution in water) was sprayed on the powder mixture and finally the granulate was dried. The GPCG-30 fluid-bed parameters used for the batches granulated at target, dry and wet condition, respectively, are listed in the tables below.

**Table 8 : Granulation conditions on GPCG-30, target condition, D**

| | Pre-warming | Granulation | Drying |
|---|---|---|---|
| Air flow | 500 m³/h | 700 > 950 m³/h | 950 > 700 m³/h |
| Spray rate | - | 200 > 250 g/min | - |
| Atomizing air flow | - | 3.2 bar | - |
| Inlet air temperature | 60°C | 50°C | 60°C |
| Outlet air temperature | 36°C (end) | 24.3°C (end) | 37°C (end) |

**Table 9 : Granulation conditions on GPCG-30, target condition, E**

| | Pre-warming | Granulation | Drying |
|---|---|---|---|
| Air flow | 500 m³/h | 700 > 950 m³/h | 950 > 700 m³/h |
| Spray rate | - | 200 > 250 g/min | - |
| Atomizing air flow | - | 3.2 bar | - |
| Inlet air temperature | 60°C | 50°C | 65°C |
| Outlet air temperature | 36°C (end) | 24.9°C (end) | 37°C (end) |

**Table 10 : Granulation conditions on GPCG-30, dry condition, F**

| | Pre-warming | Granulation | Drying |
|---|---|---|---|
| Air flow | 500 m³/h | 700 > 800 m³/h | 800 m³/h |
| Spray rate | - | 180 g/min | - |
| Atomizing air flow | - | 3.2 bar | - |
| Inlet air temperature | 60°C | 55°C | 65°C |
| Outlet air temperature | 36°C (end) | 25.7°C (end) | 37°C (end) |

**Table 11 : Granulation conditions on GPCG-30. wet condition. G**

| | Pre-warming | Granulation | Drying |
|---|---|---|---|
| Air flow | 500 m³/h | 750 > 1300 m³/h | 1050 > 850 m³/h |
| Spray rate | - | 220 g/min | - |
| Atomizing air flow | - | 3.2 bar | - |
| Inlet air temperature | 55°C | 45°C | 65°C |
| Outlet air temperature | 35°C (end) | 22.6°C (end) | 37°C (end) |

### Blending and Compression conditions

The dried granules were sieved through a hand sieve size with 0.95 mm openings and subsequently blended with external phase excipients (sieved through to 0.95 mm hand sieve) in a Gallay bin blender for 10 min at 9 rpm. In a second step, the magnesium stearate was sieved, added and blended for 5 min.

Physical characteristics of the granulates and the final blends (compression mixtures) are listed in the tables below.

**Table 12 : Physical characteristics of the granulate**

| | D target cond. | | E target cond. | F dry cond. | G wet cond. |
|---|---|---|---|---|---|
| | before sieving | after sieving | before sieving | before sieving | before sieving |
| Loose bulk volume (ml/g) | 2.16 | 2.18 | 2.20 | 2.24 | 2.08 |
| Tapped bulk volume (ml/g) | 1.98 | 1.98 | 1.99 | 2.00 | 1.91 |
| Hausner index | 1.09 | 1.10 | 1.11 | 1.12 | 1.09 |
| Carr index | 8.33 | 9.17 | 9.55 | 10.71 | 8.17 |
| Angle of repose | 37°40' | 39°30' | 39°40' | 44°20' | 36°40' |
| d50 (µ) | 318 | 313 | 302 | 265 | 393 |
| d84 (µ) | 184 | 198 | 196 | 162 | 256 |
| d84/d50 | 0.58 | 0.63 | 0.65 | 0.61 | 0.65 |
| Fraction < 75 µ (%) | 0.4 | 0.2 | 0.2 | 0.2 | 0.0 |

**Table 13 : Physical characteristics of the final blend**

| | D target cond. formula w/o aerosil | E target cond. final formula | F dry cond. final formula | G wet cond. final formula |
|---|---|---|---|---|
| Loose bulk volume (ml/g) | 2.06 | 2.08 | 2.11 | 1.98 |
| Tapped bulk volume (ml/g) | 1.80 | 1.84 | 1.88 | 1.78 |
| Hausner index | 1.14 | 1.13 | 1.12 | 1.11 |
| Carr index | 12.62 | 11.54 | 10.90 | 10.10 |
| Angle of repose | 43°20' | 36°20' (36°50')¹ | 37°40' | 35°40' |
| d50 (µ) | 318 | 263 | 244 | 332 |
| d84 (µ) | 179 | 146 | 139 | 198 |
| d84/d50 | 0.56 | 0.55 | 0.57 | 0.60 |
| Fraction < 75 µ (%) | 3.9 | 5.9 | 6.8 | 5.4 |

### Compression results

The final blend of the batches was compressed at nominal weight (1100 mg) at different compression forces and speeds on a Courtoy module S high-speed rotary tablet press (10-16 punches) using a demo punch (oval shape) set with dimension 19 x 9.5 mm. The obtained tablets were analyzed for weight, hardness, thickness, aspect, disintegration time and friability. During compression the compression settings, incl. ejection force were monitored.

The tablet cores compressed at target compression force (13N) were also coated on a lab-scale coater according to the final formulation composition (with Opadry II red at 4% level).

Despite the reasonably broad variation in GPCG-30 fluid-bed granulation conditions used, acceptable physical characteristics of the granulate and final blends are obtained in all cases (tables 12 and 13). As expected, a finer and less dense granulate is obtained when dryer thermodynamic conditions are used. Blend flowability improves with the addition of aerosil [(37°40' vs 43°20' for batches E (with aerosil) and batches D (without aerosil), respectively], confirming the functionality of the aerosil glidant material. The addition of the external phase excipients has a beneficial effect on material flowability.

Very similar physical characteristics are obtained for the granulates of batches D and E manufactured under (almost) identical granulation conditions, confirming the reproducibility of the fluid-bed granulation process.

Drying of the granulate until an outlet-air temperature of 37°C is reached results in a narrow LOD result range within 5.2 to 6.0% for the granulate and within 5.6 to 6.1 % for the final blend, confirming the reproducibility of the drying process regardless of the granulation (thermodynamic) condition used.

## Claims

1. A darunavir granulate composition consisting of darunavir or a pharmaceutically acceptable salt or solvate thereof, Hypromellose and any residual water from the granulation.

2. A darunavir granulate composition according to claim 1, wherein the darunavir is present in the form of its ethanolate and the Hypromellose is Hypromellose 2910 15 mPa.s.

3. An oral dosage form comprising about 0.4 to 0.6% by weight (w/w) of a lubricant, about 2 to 4 % by weight (w/w) of a disintegrant, microcrystalline cellulose, and about 50 to 90 % by weight (w/w) of a darunavir granulate according to claims 1 or 2, the core being optionally coated with a film coating.

4. An oral dosage form according to claim 3, wherein said core further comprises an additional active ingredient.

5. An oral dosage form according to claim 4, wherein the additional active ingredient is a cytochrome P450 inhibitor.

6. An oral dosage form according to any one of the preceding claims comprising about 0.5 % by weight (w/w) of a lubricant

7. An oral dosage form according to claim 6, comprising about 800 mg free form equivalent of darunavir.

8. A process for preparing an oral dosage form as claimed in any of the preceding claims which comprises the steps of:
- Providing granulated darunavir by; mixing water and Hypromellose 2910 15 mPa.s, spraying this first mixture on a powder of darunavir or a pharmaceutically acceptable salt or solvate thereof, and drying the so obtained darunavir granulate
- Providing a second mixture comprising microcrystalline cellulose, and a disintegrant,
- Adding granulated darunavir to the mixture and subsequent dry-blending
- Adding a lubricant and mixing until homogeneous,
- Compressing the mixture to provide the oral dosage form, said oral dosage form then being optionally film-coated.

9. An oral dosage form as claimed in any of claims 1 to 7 for use in medicine.

10. An oral dosage form as claimed in any of claims 1 to 7 for use in the treatment of HIV infection.

## Patentansprüche

1. Darunavir-Granulatzusammensetzung, welche aus Darunavir oder einem pharmazeutisch unbedenklichen Salz oder Solvat davon, Hypromellose und gegebenenfalls von der Granulierung herrührendem Restwasser besteht.

2. Darunavir-Granulatzusammensetzung nach Anspruch 1, wobei das Darunavir in Form seines Ethanolats vorliegt und es sich bei der Hypromellose um Hypromellose 2910 15 mPa.s handelt.

3. Orale Dosierungsform, welche etwa 0,4 bis 0,6 Gew.-% (w/w) eines Schmiermittels, etwa 2 bis 4 Gew.-% (w/w) eines Sprengmittels, mikrokristalline Cellulose und etwa 50 bis 90 Gew.-% (w/w) eines Darunavir-Granulats nach Anspruch 1 oder 2 umfasst, wobei der Kern gegebenenfalls mit einem Filmüberzug beschichtet ist.

4. Orale Dosierungsform nach Anspruch 3, wobei der Kern weiterhin einen zusätzlichen Wirkstoff umfasst.

5. Orale Dosierungsform nach Anspruch 4, wobei es sich bei dem zusätzlichen Wirkstoff um einen Cytochrom-P450-Inhibitor handelt.

6. Orale Dosierungsform nach einem der vorhergehenden Ansprüche, welche etwa 0,5 Gew.-% (w/w) eines Schmiermittels umfasst.

7. Orale Dosierungsform nach Anspruch 6, welche etwa 800 mg Freiformäquivalent an Darunavir umfasst.

8. Verfahren zur Herstellung einer oralen Dosierungsform nach einem der vorhergehenden Ansprüche, welches die folgenden Schritte umfasst:
- die Bereitstellung von granuliertem Darunavir durch Mischen von Wasser und Hypromellose 2910 15 mPa.s, Aufsprühen dieser ersten Mischung auf ein Pulver von Darunavir oder einem pharmazeutisch unbedenklichen Salz oder Solvat davon und Trocknen des so erhaltenen Darunavir-Granulats,
- Bereitstellung einer zweiten Mischung, die mikrokristalline Cellulose und ein Sprengmittel umfasst,
- Zugabe von granuliertem Darunavir zu der Mischung und anschließendes Trockenmischen,
- Zugabe eines Schmiermittels und Mischen bis zur Homogenität,
- Komprimieren der Mischung unter Bereitstellung der oralen Dosierungsform, wobei die orale Dosierungsform dann gegebenenfalls filmbeschichtet wird.

9. Orale Dosierungsform nach einem der Ansprüche 1 bis 7 zur Verwendung in der Medizin.

10. Orale Dosierungsform nach einem der Ansprüche 1 bis 7 zur Verwendung bei der Behandlung einer HIV-Infektion.

## Revendications

1. Composition de granulé de darunavir constituée de darunavir ou un sel pharmaceutiquement acceptable ou solvate de celui-ci, d'hypromellose et d'eau résiduelle éventuelle provenant de la granulation.

2. Composition de granulé de darunavir selon la revendication 1, dans laquelle le darunavir est présent sous la forme de son éthanolate et l'hypromellose est de l'hypromellose 2910 15 mPa.s.

3. Forme pharmaceutique orale comprenant environ 0,4 à 0,6 % en poids (m/m) d'un lubrifiant, environ 2 à 4 % en poids (m/m) d'un délitant, de la cellulose microcristalline, et environ 50 à 90 % en poids (m/m) d'un granulé de darunavir selon les revendications 1 ou 2, le noyau étant facultativement revêtu avec un pelliculage.

4. Forme pharmaceutique orale selon la revendication 3, dans laquelle ledit noyau comprend en outre une substance active additionnelle.

5. Forme pharmaceutique orale selon la revendication 4, dans laquelle ladite substance active additionnelle est un inhibiteur de cytochrome P450.

6. Forme pharmaceutique orale selon l'une quelconque des revendications précédentes comprenant environ 0,5 % en poids (m/m) d'un lubrifiant.

7. Forme pharmaceutique orale selon la revendication 6, comprenant environ 800 mg d'équivalent de forme libre de darunavir.

8. Procédé de préparation d'une forme pharmaceutique orale selon l'une quelconque des revendications précédentes qui comprend les étapes de :
- fourniture de darunavir granulé par mélange d'eau et d'hypromellose 2910 15 mPa.s, pulvérisation de ce premier mélange sur une poudre de darunavir ou un sel pharmaceutiquement acceptable ou solvate de celui-ci, et séchage du granulé de darunavir obtenu ainsi,
- fourniture d'un deuxième mélange comprenant de la cellulose microcristalline, et un délitant,
- ajout de darunavir granulé au mélange et ensuite mélange à sec,
- ajout d'un lubrifiant et mélange jusqu'à homogénéité,
- compression du mélange pour produire la forme pharmaceutique orale, ladite forme pharmaceutique orale étant ensuite facultativement pelliculée.

9. Forme pharmaceutique orale selon l'une quelconque des revendications 1 à 7 pour utilisation en médecine.

10. Forme pharmaceutique orale selon l'une quelconque des revendications 1 à 7 pour utilisation dans le traitement d'une infection par le VIH.
